Europäisches Patentamt

European Patent Office

Office européen des brevets

(11) Veröffentlichungsnummer: **0 223 944**

**A2**

# (12) EUROPÄISCHE PATENTANMELDUNG

(21) Anmeldenummer: 86112557.3

(22) Anmeldetag: 10.09.86

(51) Int. Cl.⁴: **H01R 13/506**

(30) Priorität: 25.11.85 DE 3541610

(43) Veröffentlichungstag der Anmeldung:
03.06.87 Patentblatt 87/23

(84) Benannte Vertragsstaaten:
**BE DE FR GB IT NL**

(71) Anmelder: **Grote & Hartmann GmbH & Co. KG**
**Am Kraftwerk 13**
**D-5600 Wuppertal 21(DE)**

(72) Erfinder: **Mantlik, Konrad**
**Am Eckbusch 35-5**
**D-5600 Wuppertal 1(DE)**

(74) Vertreter: **Patentanwälte Dr. Solf & Zapf**
**Schlossbleiche 20 Postfach 13 01 13**
**D-5600 Wuppertal 1(DE)**

(54) Gehäusegurt.

(57) Gehäusegurt aus Kunststoffgehäusen, die Kammern zur Aufnahme elektrischer Kontakte aufweisen, gekennzeichnet durch einen Materialsteg (4) an einem Gehäuse (2) und eine den Materialsteg (4) aufnehmende Ausnehmung (6) am anderen Gehäuse - (1) sowie ein Rastmittel für den Materialsteg (4) in der Ausnehmung (6).

FIG. 1

EP 0 223 944 A2

## Gehäusegurt

Die Erfindung betrifft einen Gurt aus Kunststoffgehäusen, die Kammern zur Aufnahme elektrischer Kontakte aufweisen.

Derartige Kunststoffgehäuse können als Einzelstücke vorliegen. Es gibt aber auch Anordnungen, bei denen mehrere Gehäuse in einer Reihe auf Abstand voneinander auf z.B. einem Klebeband lagern. Der Abstand zwischen den Gehäusen und ihre Stellung zueinander ist nicht gleich. Die Verarbeitung solcher Gehäusebänder, d.h. das Abnehmen der Gehäuse zum Bestücken mit elektrischen Kontakten erfolgt von Hand. Eine maschinelle Verarbeitung ist nicht möglich.

Des weiteren gibt es eine Anordnung, bei der die Gehäuse jeweils einteilig mit einem oder mehreren Kunststoffstegen verbunden sind. Solche Gehäusegurte sind flexibel, gewährleisten einen gleichen Abstand und eine gleiche Ausrichtung der Gehäuse, weshalb ein solcher Gehäusegurt aufspulbar und maschinell verarbeitbar ist. Dabei ist aber die Trennung eines Gehäuses vom Gurtband aufwendig. Außerdem erfordert das Gurtband zu viel Kunststoffmaterial und die Biegsamkeit des Gurtes ist relativ schlecht und beeinflußt das Auf- und Abspulen ungünstig.

Aufgabe der Erfindung ist, einen Gehäusegurt zu schaffen, der wenig Material für die Verbindung der Gehäuse erfordert, bei dem die Verbindungselemente bei der Verarbeitung nicht stören und bei dem die Gehäuse auf einfache Weise voneinander trennbar sind. Außerdem soll der Gehäusegurt biegsamer sein und weniger Trennarbeit erfordern.

Diese Aufgabe wird durch die Merkmale des Hauptanspruchs gelöst. Vorteilhafte Weiterbildungen der Erfindung werden in den Unteransprüchen gekennzeichnet.

Insbesondere vorteilhaft ist, daß die Gehäuse untereinander oder mit anderen Gehäusen, die die gleiche Verbindungseinrichtung aufweisen, nach dem Trennen wieder zusammensetzbar sind. Letzteres ist dann von Vorteil, wenn die Gehäuse für z.B. das Bestücken der Kammern mit elektrischen Kontakten vereinzelt, dann aber wieder zu einem Gurtband zusammengesetzt und weiterverarbeitet und/oder gegebenenfalls wieder aufgespult oder mit Gehäusen kombiniert werden müssen, die unterschiedliche Raumformen aufweisen und/oder an anderer Stelle mit weiteren anderen elektrischen Kontakten bestückt werden müssen. Eine solche Vielfalt von Möglichkeiten bieten die bekannten Gehäusegurte nicht. Der Stand der Tecknik gibt weder eine Anregung für die erfindungsgemäße Aufgabenstellung noch für die Lösungselemente, die die Erfindung geschaffen hat.

Anhand der Zeichnung wird die Erfindung beispielhaft näher erläutert. Es zeigen:

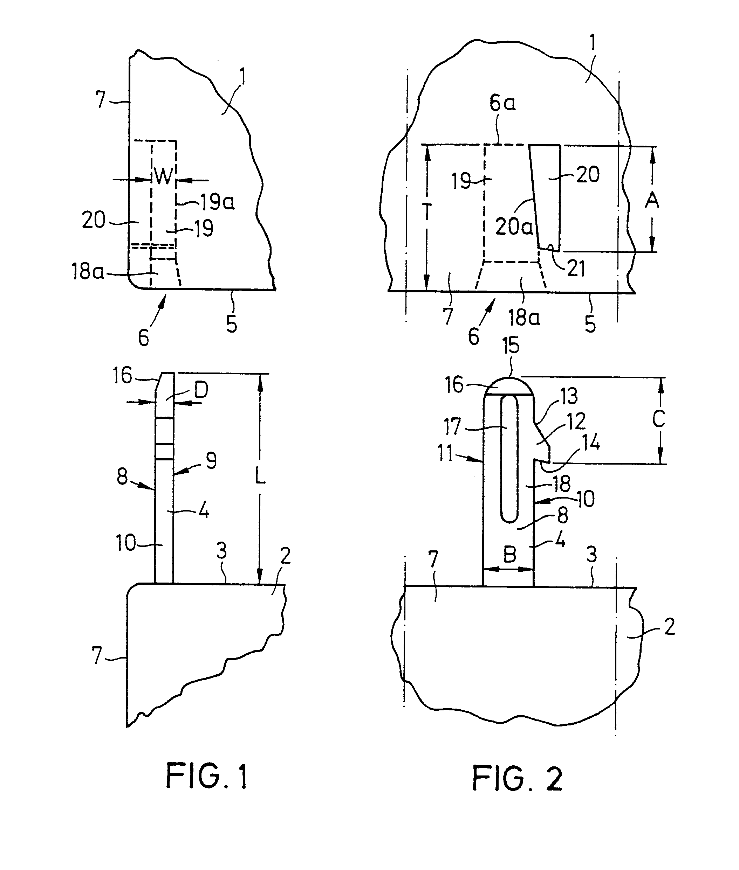

Fig. 1 oben ein Teilstück eines Unterteils eines Gehäuses und unten ein Teil eines Oberteils eines Gehäuses der erfindungsgemäßen Verbindungseinrichtung,

Fig. 2 die Teilstücke der Gehäuse nach Fig. 1 um 90° um eine vertikale Achse gedreht.

Die beiden Gehäuse 1 und 2, von denen nur Teilstücke abgebildet sind, sind zusammensteckbar ausgeführt. Zu diesem Zweck weist jedes Gehäuse 1, 2 auf z.B. einer Oberseite 3 einen senkrecht abstehenden, einstückig angespritzten Materialsteg 4 und auf z.B. der gegenüberliegenden Unterseite 5 eine Ausnehmung 6 für den Materialsteg 4 auf. Vorzugsweise befinden sich der Materialsteg 4 und die Ausnehmung 6 in der Nähe einer benachbarten Seitenfläche 7 des Gehäuses 1, 2, woraus eine gute Gelenkigkeit bzw. Biegsamkeit des aus mehreren solcher Gehäuse zusammengesetzten Gurts resultiert.

Der Materialsteg 4 ist im Querschnitt rechteckig und weist zwei Seitenflächen 8, 9 und zwei Kantenflächen 10, 11 auf. Zwischen seiner Längsmitte und dem Ende steht von der Kantenfläche 10 eine Rastnase 12 mit einer rechtwinklig von der Kantenfläche 10 abgehenden Rastkante 14 und einer davon schräg nach oben verlaufenden und in die Kantenfläche 10 einlaufenden Anlaufschräge 13 vor. Der Materialsteg 4 endet mit einem Rundbogen 15, wobei in die Seitenfläche 8 im Bereich des Rundbogens 15 eine Anfasung 16 eingebracht ist. Von der Anfasung nach unten erstreckt sich ein Langloch 17 etwa längsmittig im Materialsteg, wobei die Rastnase 12 etwa in der Längsmitte des Langlochs 17 positioniert ist. Das Langloch 17 ermöglicht dem durch das Langloch 17 gebildeten Längssteg 18 zusammen mit der Rastnase 12 ein federndes Zurückweichen in den freien Raum des Langlochs 17.

Die Ausnehmung 6 ist weiter ausgebildet, als die Dicke des Materialstegs 4 beträgt. Die Weite W verhält sich zur Dicke D z.B. wie 1,3 bis 2:1. Die Ausnehmung 6 ist im Querschnitt ebenfalls rechteckig. Ihre Tiefe T ist geringer als die Länge L des Materialstegs 4. Das Verhältnis L zu T beträgt z.B. 1,1 bis 1,4 : 1.

Die Ausnehmung 6 beginnt mit einem Findungstrichter 18a der breiter ist, als die Breite B des Materialstegs 4 beträgt. Der Findungstrichter 18a geht in einen Schacht 19 über, dessen Breite etwas breiter ist, als die Breite B des Materialstegs 4 beträgt.

Von der benachbarten Seitenfläche 7 ist ein -in der Draufsicht, also von der Seitenfläche 7 her betrachtet -viereckiges Loch 20 in den Gehäusekörper eingebracht, dessen Tiefe bis zur Rückwandung 19a des Schachts 19 reicht. Es erstreckt sich in seiner Länge vom Fuß 6a der Ausnehmung 6 bis kurz über das Ende des Findungstrichters 18a und ist seitlich versetzt zum Schacht 19 angeordnet, wobei aber die dem Schacht 19 benachbarte Seitenfront 20a des Lochs 20, die von oben nach unten schräg verläuft und die Weite des Lochs 20 verjüngt, etwas in den Schacht 19 einläuft. Das Loch 20 bildet am unteren Ende eine Raststufe 21 für die Rastkante 14 der Rastnase 12.

Der Abstand A der Raststufe 21 vom Fuß 6a des Schachts 19 bzw. der Ausnehmung 6 ist dabei größer als der Abstand C der Rastkante 14 vom Kulminationspunkt des Bogens 15 des Materialstegs 4. Das Verhältnis C zu A beträgt z.B. 1 : 1,1 bis 1,3.

Aus den angegebenen Abmessungen resultiert eine sehr gute Gelenkigkeit der Gehäuse untereinander und eine sehr gute Biegsamkeit des Gehäusegurts. Das Zusammensetzen der Gehäuse ist sehr einfach; der Materialsteg 4 wird in die Ausnehmung 6 geschoben, bis die Rastkante 14 hinter die Raststufe 21 schnappt. Das Lösen zweier Gehäuse ist ebenfalls sehr einfach. Mit einem Werkzeug kann man in das Loch 20 hineinfahren und unter Ausnutzung der Anlaufschräge 13 die Rastnase 12 zurückdrücken, bis sie außerhalb der Raststufe 21 liegt. Dann kann der Materialsteg 4 aus der Ausnehmung 6 herausgezogen werden. Das Verbinden und Lösen zweier Gehäuse ist wiederholbar, weil der Materialsteg beim Lösen sich elastisch verhält und nicht zerstört wird.

Der Materialsteg ist vorzugsweise so elastisch ausgeführt, daß die Gehäuse durch Ziehen voneinander getrennt werden können. Dabei kann von Vorteil sein, wenn die Rastkante 14 und/oder die Raststufe 21 etwas angeschrägt sind. Die Schräge verläuft z.B. bei der Rastnase 12 vom Ende der Rastnase zur Kantenfläche 10 nach unten hin. Wenn die Gehäuse fertig bearbeitet sind, kann der Materialsteg vom Gehäuse abgetrennt werden, beispielsweise durch Abschneiden oder Abbrechen.

## Ansprüche

1. Gehäusegurt aus Kunststoffgehäusen, die Kammern zur Aufnahme elektrischer Kontakte aufweisen, **gekennzeichnet durch** einen Materialsteg (4) an einem Gehäuse (2) und eine den Materialsteg (4) aufnehmende Ausnehmung (6) am anderen Gehäuse (1) sowie ein Rastmittel für den Materialsteg (4) in der Ausnehmung (6).

2. Gehäusegurt nach Anspruch 1, **dadurch gekennzeichnet,** daß sich der Materialsteg (4) und die Ausnehmung (6) in der Nähe einer benachbarten Seitenfläche (7) des Gehäuses (1, 2) befinden.

3. Gehäusegurt nach Anspruch 1 und/oder 2, **dadurch gekennzeichnet,** daß der Materialsteg (4) auf einer Kantenfläche (10) eine Rastnase (12) trägt mit einer Rastkante (14) und daß in der Ausnehmung (6) eine Raststufe (21) für die Rastkante (14) eingebracht ist.

4. Gehäusegurt nach einem oder mehreren der Ansprüche 1 bis 3, **dadurch gekennzeichnet,** daß der Materialsteg (4) im Querschnitt rechteckig ist, zwei Seitenflächen (8, 9) und zwei Kantenflächen (10, 11) aufweist und zwischen seiner Längsmitte und dem Ende von der Kantenfläche (10) die Rastnase (12) absteht, die eine schräg nach oben verlaufende und in die Kantenfläche (10) einlaufende Anlaufschräge (13) aufweist.

5. Gehäusegurt nach einem oder mehreren der Ansprüche 1 bis 4, **dadurch gekennzeichnet,** daß der Materialsteg (4) mit einem Rundbogen (15) endet, wobei in die Seitenfläche (8) im Bereich des Rundbogens (15) eine Anfasung (16) eingebracht ist.

6. Gehäusegurt nach einem oder mehreren der Ansprüche 1 bis 4, **dadurch gekennzeichnet,** daß sich von der Anfasung (16) nach unten ein Langloch erstreckt etwa längsmittig im Materialsteg, wobei die Rastnase (12) etwa in der Längsmitte des Langslochs (17) positioniert ist.

7. Gehäusegurt nach einem oder mehreren der Ansprüche 1 bis 6, **dadurch gekennzeichnet,** daß die Ausnehmung (6) weiter ausgebildet ist, als die Dicke des Materialstegs (4) beträgt, wobei sich die Weite W zur Dicke D wie 1,3 bis 2 : 1 verhält.

8. Gehäusegurt nach einem oder mehreren der Ansprüche 1 bis 7, **dadurch gekennzeichnet,** daß die Ausnehmung (6) im Querschnitt ebenfalls rechteckig ist, ihre Tiefe T geringer als die Länge L des Materialstegs ist, wobei das Verhältnis L zu T 1,1 bis 1,4 : 1 beträgt.

9. Gehäusegurt nach einem oder mehreren der Ansprüche 1 bis 8, **dadurch gekennzeichnet,** daß die Ausnehmung (6) mit einem Findungstrichter (18a) beginnt, der breiter ist, als die Breite B des Materialstegs (4) beträgt und daß der Findungstrichter (18a) in einen Schacht (19) übergeht, dessen Breite etwas breiter ist, als die Breite B des Materialstegs (4) beträgt.

10. Gehäusegurt nach einem oder mehreren der Ansprüche 1 bis 9, **dadurch gekennzeichnet,** daß von der benachbarten Seitenfläche (7) ein viereckiges Loch (20) in den Gehäusekörper eingebracht ist, dessen Tiefe bis zur Rückwandung (19a) des Schachts(19) reicht und das sich in seiner Länge vom Fuß (6a) der Ausnehmung (6) bis kurz über das Ende des Findungstrichters (18a) erstreckt.

11. Gehäusegurt nach Anspruch 10, **dadurch gekennzeichnet,** daß die dem Schacht(19) benachbarte Seitenfront (20a) des Lochs (20), die von oben nach unten schräg verläuft und die Weite des Lochs (20) verjüngt, etwas in den Schacht (19) einläuft.

12. Gehäusegurt nach Anspruch 10 und/oder 11, **dadurch gekennzeichnet,** daß das Loch (20) am unteren Ende die Raststufe (21) für die Rastkante (14) der Rastnase (12) bildet.

13. Gehäusegurt nach einem oder mehreren der Ansprüche 10 bis 12, **dadurch gekennzeichnet,** daß der Abstand A der Raststufe (21) vom Fuß (6a) des Schachts (19) größer als der Abstand C der Rastkante (14) vom Kulminationspunkt des Bogens (15) des Materialstegs (4) ist, wobei das Verhältnis C : A wie 1 : 1,1 bis 1,3 beträgt.

14. Gehäusegurt nach einem oder mehreren der Ansprüche 1 bis 13, **dadurch gekennzeichnet,** daß die Rastkante (14) und/oder die Raststufe (21) etwas angeschrägt sind, wobei die Schräge bei der Rastnase (12) vom Ende der Rastnase zur Kantenfläche (10) nach unten hin verläuft.

FIG. 1

FIG. 2

4442